(19)

Europäisches Patentamt
European Patent Office
Office européen des brevets

(11) **EP 4 480 391 A1**

(12) **EUROPEAN PATENT APPLICATION**

(43) Date of publication:
**25.12.2024 Bulletin 2024/52**

(21) Application number: **23461609.2**

(22) Date of filing: **23.06.2023**

(51) International Patent Classification (IPC):
**A61B 3/14** *(2006.01)* **A61B 3/11** *(2006.01)*

(52) Cooperative Patent Classification (CPC):
**A61B 3/112; A61B 3/14**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Solvemed Group sp. z o.o.**
**61-806 Poznan (PL)**

(72) Inventors:
- **Bogucki, Aleksander**
  **01-003 Warszawa (PL)**
- **Chrapkiewicz, Radoslaw**
  **Sunnyvale, CA 94087 (US)**
- **Chrost, Hugo**
  **64-100 Leszno (PL)**
- **Jachura, Micha**
  **02-353 Warszawa (PL)**

(74) Representative: **AOMB Polska Sp. z.o.o.**
**ul. Rondo Ignacego Daszynskiego 1**
**00-843 Warsaw (PL)**

(54) **A METHOD FOR REDUCING THE VARIABILITY OF PUPILLARY LIGHT RESPONSE IN A VISIBLE-LIGHT PUPILLOMETRY**

(57)     The subject matter of the present invention is a method for external light adjusted pupillometry measurement. Moreover, the invention relates to a method for reducing the variability of pupillary light response in visible-light pupillometry implemented with the use of a diagnostic device in non-controlled environmental conditions. The variability is reduced by calculating and using optimal intensity of stimulus light based on light sensor settings, which keeps the stimulation proportional to the external light already illuminating the subject's eye. Optionally a pre-recording quality checks are introduced to make sure that the device operates within the designed acceptable range of external light and distance between the diagnostic device and the subject's eye. An optional post-recording quality checks are also proposed which confirm the quality of collected data and make sure that external conditions haven't changed during the measurement.

Fig. 2

**Description**

**Technical field**

The present disclosure relates to the field of medicine, ophthalmology, neuro-ophthalmology, neurology with a subfield of pupillometry specializing in determination of a pupil light response reflecting the subject medical condition. The subject matter of the invention is a method for bringing into conformity pupillary light response performed in visible-light pupillometry performed using a single diagnostic or measurement device, especially in non-controlled environmental conditions such as the intensity of external light and camera-to-eye distance.

**Prior art**

In the state of the art, a variety of pupil measurement systems and methods are reported. For instance, patent document published in 2018 [WO2018222897A1] proposes a technique employing machine learning to enable smartphone-based visible light pupillometry. A smartphone, that is a device which might be used to record a visible light video of a pupillary light reflex (PLR) and afterwards a machine learning model might be used to retrieve a size of a pupil along with its diameter change over time. The solution requires a specially designed box which simultaneously reduces external illumination of the eye and controls the distance between the subject's face and the camera so that pixel sizes may be converted into absolute measurement sizes, i.e., expressed in physical units such as inches or millimetres. In some embodiments instead of the aforementioned box the authors propose that the subject will hold an object of known size during the measurement, such as an ID card, drivers' license, coin, etc., next to the eye in order to translate the pupil image pixel size into actual dimensions. Thanks to the device-to-eye distance control and external illumination separation there is no need to bring into conformity the measurements made by a single device under different environmental conditions.

Another patent document published in 2018 [US10070787B2] described a method for detecting and monitoring pupillary light reflex (PLR) responses and physical conditions using a portable video capture device for the purpose of diagnosing or monitoring impairment due to consumption of intoxicating substance or neurological disorder due to trauma or illness. A method of testing a user for impairment relies on a portable device in an opaque enclosure that opaque enclosure controls light conditions by substantially eliminating external light, thereby providing complete or near darkness for the eyes. Similarly, this avoids the problem of making the measurements under variable environmental conditions such as external illumination or camera-to-eye distance.

A patent document published in 2017 [US10034605B2] describes a method for detection of brain trauma providing a light stimulus to an individual's eye and tracking an optical response of the individual's eye. More specifically, in one embodiment, a method for determining risk of brain trauma, includes employing a pupillometry device which includes: a digital camera, a light element, a user interface, a computer processor, and computer memory. A user or patient orients a patient's eye to receive external light, and orients the digital camera to view the patient's pupil. The pupillometry device then emits a flash of light to the patient's pupil, captures a series of images of the patient's pupil, and determines the location and relative size of the patient's pupil in each image. The pupillometry device then determines, from the series of images and the relative size of the patient's pupil in each image, one or more rates of change of the size of the patient's pupil, and compares the rates of change of the size of the patient's pupil to one or more baseline rates of change to determine a risk level of brain trauma to the patient based on the deviation of the rates of change of the size of the patient's pupil from the one or more baseline rates of change. The device then indicates the risk level of brain trauma. This method does not include any mean of taking into account measurement environment thus the measurement results yield by the device might be affected e.g., by external illumination conditions, camera-to-eye distance or eye focusing point of the diagnosed subject.

A patent document published in 2021 [WO2021034951A1] describes a systems and methods for measuring pupillary responses to visible light stimuli. The system provides a display and a camera on the same side of a device; the display provides a visible light stimulus to illuminate a subject's face and cause a pupillary reflex of an eye. A visible light or infrared camera thereafter receives image data of the pupillary light reflex. In some examples, an external light sensor and infrared measurement systems allow for performing pupillary light reflex assessments in low lighting conditions. In some embodiments of the invention the external light measurement is used to determine: (i) whether the external illumination is sufficient to provide image data of adequate quality; (ii) whether the illumination requires an infrared sensing system to capture images of the pupils with sufficient quality to process them and identify various pupillary features disclosed; (iii) whether it is possible to utilize a display to provide a visible light stimulus, so that the contrast between the lux of the visible light stimulus emitted by the display and the external light lux is sufficient to trigger a PLR.

Patent document published in 2021 [US0000339A1] reveals pupillometry systems, methods and devices. The pupillometry systems include at least one camera, at least one radiation source configured to project radiation to the one or more pupils, a computer system in data communication, the computer system having a processor and a non-transitory computer-readable storage medium. The non-transitory computer-readable storage medium includes computer-readable instructions for collecting and time stamping the image data, processing the raw image data in such a way as to bring the images into conformance with standardized image parameters, identifying and measuring the one or more pupils in the image data, processing the image data to produce measurement data of change in the one or more pupillary characteristics, calculating a standardized output of measurement data for the one or more pupillary characteristics, and providing a mechanism to share or store this information with other users via a computer network. The system may provide several adjustments to improve pupillometry measurement. The adjustment of the image properties and/or adjustment of the criteria used to identify the pupil is based on measurements of the existing external illumination, for instance: (i) adjusting the parameters used to identify the pupil based on known properties of the imaging device or analysis of images obtained from the imaging device, including, but not limited to, image brightness, contrast, frame rate, magnification, distribution of pixel intensities in one or more colour channels; (ii) adjusting the intensity of the radiation source based on internally stored data that enumerates recommended values for different types of radiation sources; (iii) adjusting the brightness, contrast, zoom, focus, and other camera properties based on internally stored data that enumerates recommended values for different types of cameras. The purpose of the above adjustments may be to increase the accuracy of the identification of the pupil and/or iris by the pupillometer, or to bring the measurements into conformance with captures made by devices with different types of cameras or radiation sources or external light conditions, or both.

A US patent document published in 2017 [US10506165B2], describes a concussion screening device which displays stimuli on the illumination unit and receives a plurality of images on the image sensor array. The stimuli correspond to a concussion test. The processor processes the plurality of images, which includes detecting one or more pupils of an evaluated person during the concussion test. Based on the processing, the concussion screening device determines whether or not an evaluated person has suffered a concussion. A device provides a guidance to a user in positioning the concussion diagnostic device relative to a person to obtain a focal distance, the positioning based on a distance determined by the range finder. Thanks to the embedded light sensor the device also provides guidance to move to a lower lighting environment when the light sensor detects an external light intensity above a brightness threshold where pupils constrict to the point where pupil detection is unreliable or impossible.

Another patent document published in 2021 [US11003936B2] discloses a method and system for controlling an eye tracking system. The system includes non-transitory computer-readable storage medium for controlling an eye tracking setup to optimize eye tracking performance under different lighting conditions, by obtaining a first image captured using a camera associated with the eye tracking system. The said first image comprising at least part of an iris and at least part of a pupil of an eye illuminated by an illuminator associated with the eye tracking system at a current power of illumination selected from a set of predetermined power levels. The system determines a contrast value between the iris and the pupil in the image, and if the contrast value deviates less than a pre-set deviation threshold value from a pre-set minimum contrast value, the system sets the current power of illumination of the illuminator to the other predetermined power level in the set of predetermined power levels.

**Problem from the state of the art**

In the prior art there exists pervasive problem of the sensitivity of measured pupillometric parameters with respect to external illumination conditions during the pupil light reflex measurement which results in a significant variability of the results obtained in various environmental conditions. This variability is even more pronounced when the distance between stimulation light source and the subject's eye is uncontrolled resulting in stimulation light intensity changing from measurement to measurement. Increased variability of pupillometric parameters increases the uncertainty of reaction assessment and renders the measurements useless for diagnostic purposes. Thus, the reduction of variability of pupillary light response in visible-light pupillometry in non-controlled environmental conditions is a highly desirable and anticipated functionality.

**Solution to the problem**

In the described method the intensity of external light illuminating the subject's eye is estimated by reading out the settings of camera light sensor. Furthermore, the distance from camera-to-eye is retrieved from the position of focusing element within the camera imaging system or from the iris size which diameter distribution among population is narrow. Based on the above parameters the optimal intensity of stimulus light is calculated to keep the stimulation proportional to the light already illuminating the subject's face. In order to observe the most pronounced pupil reaction the proportionality factor is the highest possible, while accounting for the finite intensity of used stimulus light source and for the expected range of external light. Optimal intensities of stimulus light calculated for specific environmental conditions using this approach ensure that the pupillary light response is similar over the broad range of non-controlled environmental conditions.

**Glossary and definitions of terms known in the state of the art and used in the patent specification**

Throughout the present specification (both above and below the glossary section), the terms used have the following meanings described in the definitions below:
The term "ACV" is a pupillometric parameter describing the average velocity of pupil constriction induced by the stimulus light. The parameter is usually expressed in millimetres/second.

The term "ADV" is a pupillometric parameter describing the average velocity of pupil dilation induced by turning off the stimulus light. The parameter is usually expressed in millimetres/second.

The term "CAMP" is a pupillometric parameter describing the amplitude of pupil constriction induced by the stimulus light. The parameter is usually expressed in millimetres.

The term "diagnostic device" or alternatively "measurement device" means electronic device equipped with a central processing unit, a memory unit, a light sensor, a light source and optical imaging system with adjustable focusing distance, which can capture an image. This includes a dedicated stand-alone device, a digital camera, portable video device, or a smartphone equipped with one or several cameras. The said optical imaging system with adjustable focusing distance contains a plurality of lenses.

The term "external light sensor" means light detector which might be embedded in diagnostic device for the purpose of measuring the level of external light.

The term "visible light" means a part of electromagnetic light spectrum which can be detected by the eye of the measured subject such as human or animal and thus induce pupillary light response.

The term "exposure time - Q" means the setting of light sensor which defines the exposition time $\Omega$ of each video frame. Usually, exposure time values ranging from below milliseconds up to tens of milliseconds are used.

The term "exposure value" also referred to as EV is an indicator of camera settings, corresponding to actual combination of exposure time, aperture size and ISO setting such that all combinations of aforementioned parameters that yield the same exposure conditions result in the same EV of the captured image. The term exposure value is of a broad use in photography.

The term "external light" means total light illuminating the pupil independently from stimulus light source. Sources of the external light might include room lighting, day light or any other source which is not controlled by the diagnostic device. Non-controlled environmental conditions such as changing intensity of external light affects the initial pupil diameter and in consequence increases the variability of pupillary light response in visible-light pupillometry performed by a diagnostic device.

The term "focusing element" means a moveable plurality of lenses (or lens assembly) or a single lens which is a part of camera imaging system and is responsible for setting the focusing distance of the camera.

The variable "x" stands for the distance between the diagnostic device and the subject's eye.

The term "ISO" means the setting of light sensor which modifies its sensitivity to capturing light. Higher ISO values correspond to high sensor sensitivity allowing for video capture in low external light conditions, whereas lower ISO values correspond to low sensor sensitivity allowing for video capture in high external light conditions. The parameter is a digital equivalent of an exposure index used for the characterisation of analogue photographic film.

The term "light sensor" means a CCD image sensor, EMCCD image sensor, CMOS image sensor, sCMOS sensor or other optoelectronic device containing an array of light sensitive pixels used for creating digital images.

The term "MCV" is a pupillometric parameter describing the maximum velocity of pupil constriction induced by the stimulus light. The parameter is usually expressed in millimetres/second.

The term "optimal stimulus light power" means a stimulus light power which increases monotonically in response to increasing external light. In particular it could be proportional to the intensity of external light with a proportionality factor $\gamma$.

The term "PDV" is a pupillometric parameter describing the maximal (peak) velocity of pupil dilation induced by turning off the stimulus light. The parameter is usually expressed in millimetres/second.

The term "proportionality factor y" means a ratio of stimulus to external light intensity expressed in a form of unitless number. As an example, when the intensity of stimulus light is equal to the intensity of external light the proportionality factor $\gamma$ equals one.

The term "region of interest" is a selected part of an image which provides a basis for the exposure setting algorithm.

The term "stimulus light" means a light emitted from a light source that is generated by the diagnostic device in order to induce pupillary light reaction. A level of a stimulus light is controlled by the diagnostic device.

The term "pre-recording quality check" means an additional and optional step in the invented method which verifies if data acquisition parameters are within acceptable range and will result in the correct pupillogram curve measurement. During the pre-recording quality check a feedback information might be given to the diagnostic device operator to modify the external conditions before the start of the measurement or to tilt/move the diagnostic device before the start of the measurement.

The term "post-recording quality check" means an additional and optional step in the invented method which verifies if the quality of collected video (such as the sharpness and the brightness) is within the acceptable range. During the post-recording quality check a feedback information might be given to the diagnostic device operator to repeat the measurement.

The term "pupillary light response" or "pupillary light reflex" is an automatic physiological response which optimizes light reaching the retina by controlling the pupil diameter in response to the intensity (luminance) of light that falls on the retinal ganglion cells of the retina. The contraction or dilation of the pupil is realized using the iris sphincter.

**Summary of the invention**

The invention relates to a method for reducing the variability of pupillary light response in visible-light pupillometry implemented with the use of a mobile diagnostic device comprising a digital camera, an internal light source and a control unit, wherein the internal light source emits stimulus light in a visible spectrum with variable intensity based on the calculations of the control unit, wherein collected measured data are vulnerable to non-controllable external environmental conditions and stimulus light intensity emitted from the internal light source depends on camera light sensor settings and the camera-to-eye distance, said method comprising the following steps:

a. the camera light sensor settings including sensor sensitivity ISO and sensor exposure time $\Omega$ are adjusted to yield the exposure value EV of the captured video in the range between the minimum exposure value and the maximum exposure value [$EV_{min}$, $EV_{max}$], wherein the values of ISO and $\Omega$ yielding the expected exposure value are retrieved and stored for further use;

b. the camera-to-eye distance $x$ is retrieved from at least one of the following data: (i) the position of focusing element within the camera imaging system; and/or (ii) the subject's face size in the captured video; and/or (iii) the measurement of the iris diameter; and/or (iv) the parallax effect estimated for images captured by different cameras; and/or (v) dedicated distance sensor based on ultrasound waves, infrared light, or LIDAR technique;

c. the optimal stimulus light intensity $L$ is calculated by the control unit using the function $L(x)$ or $L(ISO, \Omega)$ or $L(ISO, \Omega, x)$, wherein the exact values of function arguments are retrieved from the steps a and b, while the optimal stimulus light intensity L is either expressed in luminance units or in optimal stimulus light power varying from 0% to 100% with the latter case referred to as $L_{pow}(x)$, $L_{pow}(ISO, \Omega)$ and $L_{pow}(ISO, \Omega, x)$ respectively.

The method according to the invention is especially suitable to be implemented in non-controlled environmental conditions and has advantages over prior art methods implemented in such conditions.

Preferably, the inventive method further comprises step:

d. a video of an eye is captured, including the time period before the eye is illuminated with the optimal stimulus light intensity calculated in step c, the time period when the eye is illuminated with said stimulus and the time period after the eye is illuminated with said stimulus, and afterwards a pupillometric curve is reconstructed by feeding the measured video frame-by-frame data to image segmentation algorithm learned beforehand by means of an annotated dataset.

Preferably, the optimal stimulus light intensity $L$ calculated using the function $L(x)$ or $L(ISO, \Omega)$ or $L(ISO, \Omega, x)$ or alternatively $L_{pow}(x)$, $L_{pow}(ISO, \Omega)$ or $L_{pow}(ISO, \Omega, x)$, respectively, increases monotonically in response to increasing external light.

Preferably, the optimal stimulus light intensity $L$ calculated using the function $L(x)$ or $L(ISO, \Omega)$ or $L(ISO, \Omega, x)$ or alternatively $L_{pow}(x)$, $L_{pow}(ISO, \Omega)$ or $L_{pow}(ISO, \Omega, x)$, respectively, is proportional to the intensity of external light with a proportionality factor $\gamma$.

Preferably, the settings of the camera light sensor which yield the exposure value EV of the captured video in the range of [$EV_{min}$, $EV_{max}$] are based on either the entire image or the selected region of interest within said image, such as the area of subject's iris, sclera or forehead.

Preferably, the settings of the camera light sensor which yield the exposure value EV of the captured video in the range of [$EV_{min}$, $EV_{max}$] are adjusted manually by a diagnostic device operator or automatically by an auto-exposure algorithm.

Preferably, a family of functions $L_n(ISO, \Omega, x)$ is used for the calculation of the optimal stimulus light intensity $L$ with a specific function selection depending on the iris colour, skin colour or hair colour of the measured subject.

Preferably, the values of the function $L(ISO, \Omega, x)$ or the family of functions $L_n(ISO, \Omega, x)$ are pre-calculated and stored on a computer readable storage medium.

Preferably, the diagnostic device is used, which is equipped with a dedicated light sensor, whose readout $I_{ext}$ is used to calculate optimal stimulus light intensity $L$ by using the function $L(x)$ or $L_{pow}(x)$, respectively, which additionally depends also on $I_{ext}$.

Preferably, the range [EV$_{min}$, EV$_{max}$] is defined as [$\eta$ -0.5, $\eta$+0.5], where $\eta$ is a real number belonging to the interval [-6, 21].

Preferably, the range [EV$_{min}$, EV$_{max}$] is defined as [$\eta$-0.1, $\eta$+0.1], where $\eta$ is a real number belonging to the interval [-6, 21].

Preferably, the optimal stimulus light power is calculated using a plot $L_{pow}(x)$ or $L_{pow}(ISO, \Omega)$ or $L_{pow}(ISO, \Omega, x)$, wherein the function $L_{pow}(x)$ or $L_{pow}(ISO, \Omega)$ or $L_{pow}(ISO, \Omega, x)$ is defined implicitly as a numerical solution to the nonlinear equation:

$$\left[ Lum\left( x, \ L_{pow}(x) \right) - Lum(x, \ p_0) \right] = \gamma \cdot \left( \frac{\Delta}{ISO \cdot \Omega} + Lum(x, \ p_0) \right)$$

Or

$$\left[ Lum\left( x, \ L_{pow}(ISO, \ \Omega) \right) - Lum(x, \ p_0) \right] = \gamma \cdot \left( \frac{\Delta}{ISO \cdot \Omega} + Lum(x, \ p_0) \right)$$

Or

$$\left[ Lum\left( x, \ L_{pow}(ISO, \ \Omega, \ x) \right) - Lum(x, \ p_0) \right] = \gamma \cdot \left( \frac{\Delta}{ISO \cdot \Omega} + Lum(x, \ p_0) \right)$$

Preferably, after the steps a and b a pre-recording quality check is performed based on the values of parameters $(ISO, \Omega, x)$ retrieved in these steps and if the values of said parameters are out of a predefined range - feedback information is given to a diagnostic device operator to modify the external conditions before the start of the measurement.

Preferably, an additional pre-recording quality check is performed based on the diameter of the pupil in the video and the position of the light source reflection on the eyeball and if these image features are out of the predefined range - feedback information is given to an operator to tilt/pan or move the diagnostic device before the start of the measurement.

Preferably, after completing the method steps an additional post-recording quality check is performed based on the sharpness and brightness of the video or pupil diameter measurement uncertainty and if these parameters are out of predefined range - feedback information is given to an operator to repeat the measurement.

## Advantageous effects of the invention

The invention according to the present patent specification has numerous advantages comparing to the known solutions in the prior art. First of all, the invented method reduces the well-known vulnerability of visible-light pupillometry to the non-controlled environmental conditions by maintaining the stimulation light intensity proportional to the light already illuminating subject's eye. It also adaptively adjusts the stimulus light power to the distance between the diagnostic device and the subject's eye. Furthermore, the proposed pre-recording quality checks make sure that the device operates within the designed acceptable range while the post-recording quality checks reduce the number of potential outliers in the collected data.

## Possible areas of application of the invention

The present invention finds a primary use in a broad field of pupillometry, ophthalmology and neurological studies. A pupillary light response is nowadays studied within the context of other technological fields, such as medicine, automotive, virtual reality, gaming, education, quick drug detection in law enforcement etc. The visible light pupillometry performed is prone to the variability resulting from non-controlled light measurements conditions. The demonstrated reduction of pupil light reaction variability might play a crucial role in increasing a sensitivity of prospective algorithms designed for neurodegenerative disorders diagnostics and tracking their progress.

## Preferred embodiments of the invention

Now, the invention will be presented in more detail, in a preferred embodiment, with reference to the attached drawing in which:

Fig. 1 Shows a luminance of stimulus light source $Lum(x,p)$ depending on its power level $p$ and distance from the source $x$. The stimulus light source characterized by the presented calibration curves has been used in all presented embodiments of the invention.

Fig. 2 Left panel (a) shows a measurement scheme illustrating a method for reducing the variability of pupillary light response in visible-light pupillometry resulting from non-controlled device-to-eye distance. Additionally, right panel (b) presents the graphical representation of measured CAMP parameter

Fig. 3 Presents an optimal stimulus light power in a form a plot $L_{pow}(x)$

Fig. 4 Left panel shows a measurement scheme illustrating a method for reducing the variability of pupillary light response in visible-light pupillometry resulting from non-controlled external light. Additionally, right panel presents the graphical representation of measured CAMP, ADV, ACV, MCV, and PDV parameters.

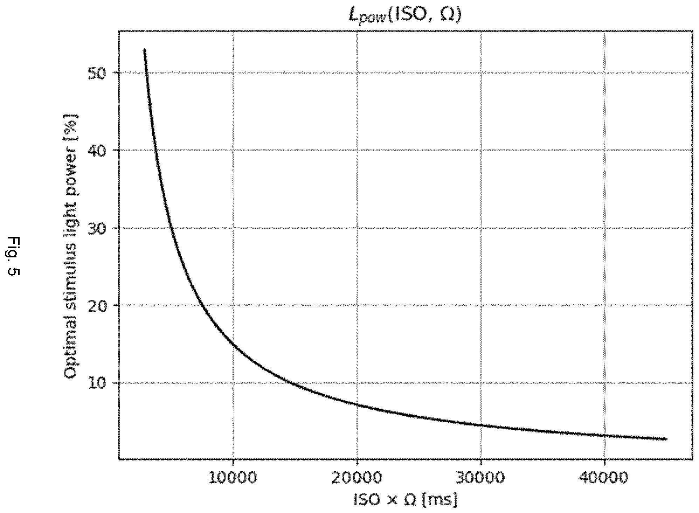

Fig. 5 Presents an optimal stimulus light power in a form a plot $L_{pow}(ISO, \Omega)$

Fig. 6 Presents a series of pupilograms measured in uncontrolled external light conditions (varying from 10 lux to 160 lux) using either fixed stimulus light intensity of 340 lux (corresponding to 50% of available stimulus light power) or using optimal stimulus light power.

Fig. 7 Presents an average of pupilogram series measured in uncontrolled external light conditions (varying from 10 lux to 160 lux) using either fixed stimulus light intensity of 340 lux (corresponding to 50% of available stimulus light power) or using optimal stimulus light power. The uncertainty of the curve has been characterized as a standard deviation resulting from measurement series. It has been presented as a shaded region above and below the pupillogram curve.

Fig. 8 Left panel (a) shows a measurement scheme illustrating a method for reducing the variability of pupillary light response in visible-light pupillometry resulting from both non-controlled device-to-eye distance as well as non-controlled external light. Additionally, right panel (b) presents the graphical representation of measured CAMP parameter

Fig. 9 Presents an optimal stimulus light illuminance in a form of a matrix plot $L (ISO, \Omega, x)$

Fig. 10 Presents an optimal stimulus light power in a form of a matrix plot $L_{pow}(ISO, \Omega, x)$

Fig. 11 Presents an average of pupilogram series measured in uncontrolled external light conditions (varying from 10 lux to 160 lux) and uncontrolled camera-to-face distance (varying from 100 mm to 200 mm) using either fixed stimulus light intensity of 340 lux (corresponding to 50% of available stimulus light power) or using optimal stimulus light power. The uncertainty of the curve has been characterized as a standard deviation resulting from measurement series. It has been presented as a shaded region above and below the pupillogram curve.

Fig. 12 Presents a scheme of quality checks which might be applied to additionally reduce the variability of measured pupil light response.

The figure uses notation that is consistent with the glossary definitions.

**Embodiments of the invention**

The following examples are included only to illustrate the invention and to explain particular aspects thereof, and not to limit it, and should not be interpreted as the entire range thereof which is defined in the appended patent claims. In the following embodiments, unless indicated otherwise, standard materials and methods used in the technical field were used or manufacturers' recommendations for specific devices, materials and methods were followed. It must be noted that, as used in the described embodiments and the appended claims, the singular forms "a," "an," and "the" include plural referents unless the context clearly dictates otherwise.

**EMBODIMENT 1**

**A method for reducing the variability of pupillary light response in visible-light pupillometry resulting from non-controlled device-to-eye distance**

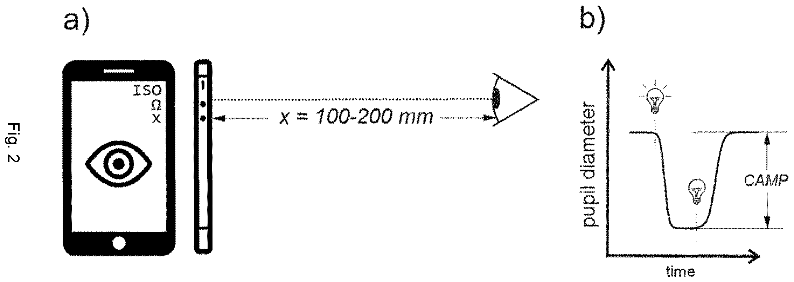

In the following embodiment a smartphone (Apple iPhone 13 Pro) is selected as a diagnostic or measurement device comprising a digital camera with an internal visible light source. The device is employed to collect the videos of pupillary light response induced by embedded flashlight with the video frame rate of 60 frames per second. After video capture each frame is fed into image segmentation or detection algorithm, estimating the pupil diameter and the iris diameter. Beforehand the image segmentation algorithm has been trained using an annotated dataset. The CAMP parameter is retrieved from the dynamics of pupillary light response also known as a pupillogram curve (see Fig. 2b).

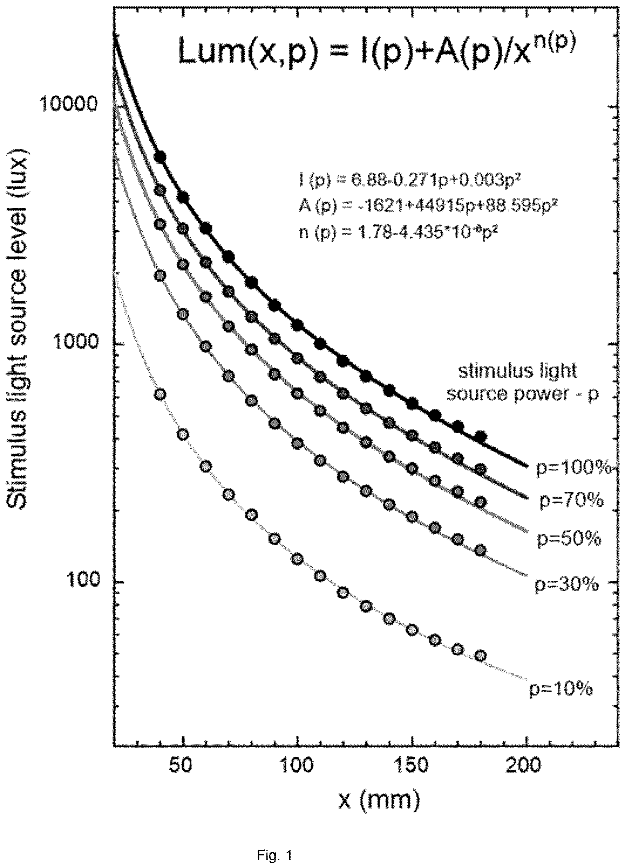

The reduction of the variability of pupillary light response is realised by adjusting the stimulus intensity to the distance between the smartphone and the subject's eye. The detailed calibration curve of stimulus light source has been presented in Fig. 1. Before starting the measurement of pupillary light response, the settings of the light sensor are selected by the auto-exposure algorithm of the smartphone in order to bring the exposure value EV close to zero. The region of interest of the auto-exposure algorithm is the entire image. The values of ISO and $\Omega$ yielding the desired exposure value (EV) were retrieved and their values yielded ISO = 2200 and $\Omega$ = 16 milliseconds. The device-to-eye distance has been retrieved from the pre-calibrated position of focusing element within the camera imaging system. The measurement scheme is presented in Fig. 2(a). Alternatively, the camera-to-eye distance $x$ is retrieved from at least one of the following data: (i) the position of focusing element within the camera imaging system; and/or (ii) the subject's face size in the captured video; and/or (iii) the measurement of the iris diameter; and/or (iv) the parallax effect estimated for images captured by different cameras; and/or (v) dedicated distance sensor based on ultrasound waves, infrared light, or LIDAR technique.

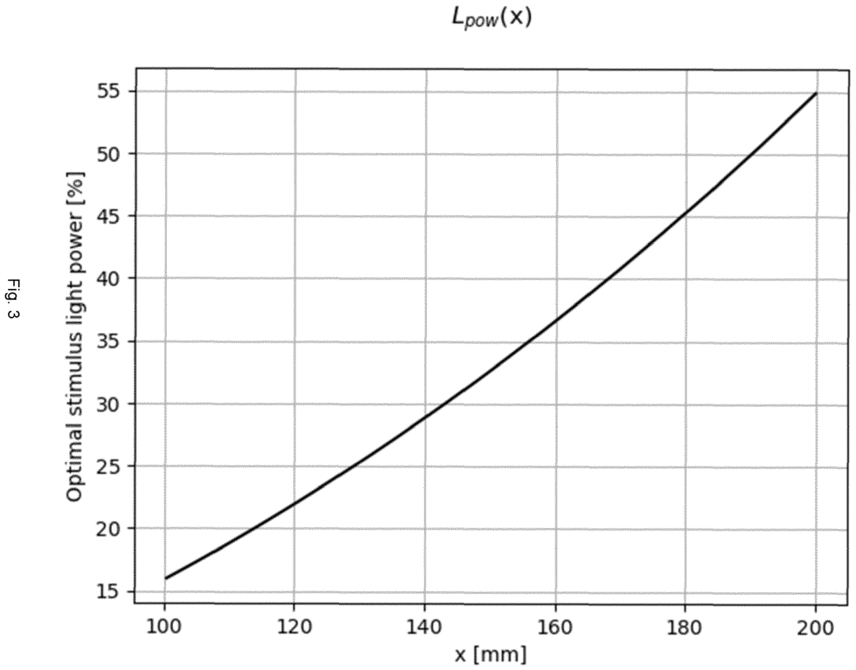

In order to confirm the reduction of variability a series of measurements of CAMP parameter was made which is the amplitude of pupil contraction induced by the stimulus light expressed in millimetres. The definition of CAMP has been graphically presented in Fig. 2(b). The CAMP parameter of the volunteer eye has been measured for a plurality of distances between volunteer's eye and the diagnostic device: 100 mm, 150 mm and 200 mm. Since the embodiment is aimed at reducing the variability resulting from non-controlled device-to-eye distance the measurements were made in a controlled external light conditions yielding similar ISO and $\Omega$ values for all measurement series. In the first measurement series the power of stimulus light intensity was always fixed and selected as 100% corresponding to the stimulus light luminance of 1190 lux, 570 lux and 340 lux, respectively depending on the selected camera-to-eye distance (100 mm, 150 mm and 200 mm). In the second measurement series an optimal stimulus light power is calculated using a plot $L_{pow}(x)$ presented in Fig. 3. The function $L_{pow}(x)$ is defined implicitly as a numerical solution to the nonlinear equation:

$$\left[Lum\left(x, L_{pow}(x)\right) - Lum(x,\, p_0)\right] = \gamma \cdot \left(\frac{\Delta}{ISO \cdot \Omega} + Lum(x,\, p_0)\right), \quad (1)$$

where:

- $Lum(x,p)$ is a luminance of stimulus light source depending on its power level $p$ and distance from the source $x$ presented in Fig.1 (in the discussed embodiment $x$ = 100 mm, 150 mm or 200 mm).
- $ISO, \Omega$ are light sensor parameters ISO and exposure time respectively.
- $\gamma$ is the proportionality factor of the stimulation (in the discussed embodiment $\gamma$ = 20).
- $\Delta$ is the calibration constant relating light sensor settings to external light (in the discussed embodiment the measured constant $\Delta$ equals 455 s).
- $p_0$ is a power level of a stimulus light source before and after the stimulation (in the discussed embodiment $p_0$ = 0%) which might be optionally increased to facilitate pupil and iris detection

The left-hand side of the equation defines the stimulus light resulting from switching the power level of the light stimulus source from $p_0$ to $L_{pow}(x)$. The right-hand side of the equation is the external light reaching the subject's face before the stimulation multiplied by the proportionality factor $\gamma$. Thus, the formula ensures that the stimulation inducing the pupil light reaction is proportional to the light already illuminating the subject's pupil. The choice of specific proportionality factor $\gamma$ depends on the available optical power of the used stimulus light source as well as from the expected external light range.

The measurements were performed in the external light of 10 lux, while the optimal stimulus light power has been selected to maintain the stimulation at the level of 200 lux. Hence the proportionality factor $\gamma$ in the optimized stimulus measurement series equalled 20.

Table 1. A set of CAMP parameters measured for uncontrolled distance between the diagnostic device and the subject's eye using either fixed stimulus light power or optimal stimulus light power.

| CAMP | x = 100 mm | x = 150 mm | x = 200 mm |
|---|---|---|---|
| Stimulus light power 100% | 2.56 mm | 2.11 mm | 1.96 mm |
| Optimal stimulus light power | 1.98 mm | 1.87 mm | 1.91 mm |

The CAMP parameter measurements for fixed stimulus light power and optimal stimulus light power are presented in Table 1. The fixed stimulus pupillary light response varied from 1.96 mm to 2.56 mm for the measurements performed with x varying from 200 mm to 100 mm (resulting in 31% of relative CAMP change). The optimal stimulus pupillary light response varied from 1.87 mm to 1.98 mm for the measurements performed with x varying from 200 mm to 100 mm (resulting in 6% of relative CAMP change). This is presenting the expected reduction of pupillary light response variability resulting from non-controlled device-to-eye distance.

**EMBODIMENT 2**

**A method for reducing the variability of pupillary light response in visible-light pupillometry performed by a smartphone resulting from non-controlled external light**

In the following embodiment a smartphone (Apple iPhone 13 Pro) is selected as a diagnostic or measurement device comprising a digital camera with an internal visible light source. The device is employed to collect the videos of pupillary light response induced by embedded flashlight with the video frame rate of 60 frames per second. After video capture each frame is fed into image segmentation or detection algorithm, estimating the pupil diameter and the iris diameter. Beforehand the image segmentation algorithm has been trained using an annotated dataset. The canonical pupillometric parameters such as CAMP, MCV, PDV, ACV, ADV are retrieved from the dynamics of pupillary light response also known as a pupillogram curve (see Fig. 2b).

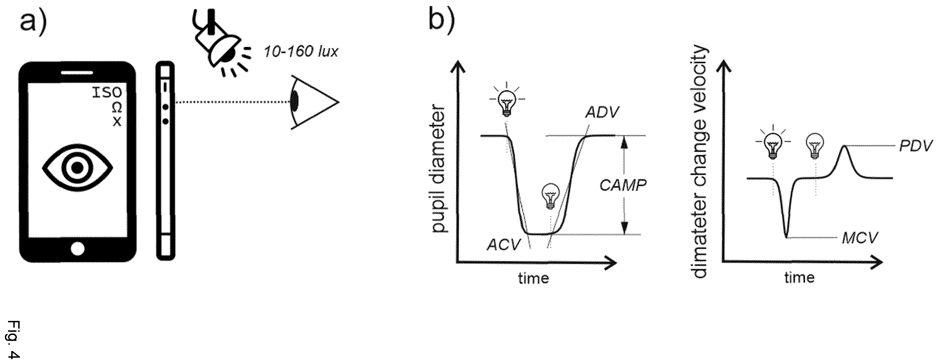

The reduction of the variability of pupillary light response is realised by adjusting the stimulus light intensity to the external light intensity. The detailed calibration curve of stimulus light source has been presented in Fig. 1. Before starting the measurement of pupillary light response, the settings of the light sensor are selected by the auto-exposure algorithm of the smartphone in order to bring the exposure value EV close to zero. The region of interest of the auto-exposure algorithm is the entire image. The values of ISO and $\Omega$ yielding the desired exposure value were retrieved and their values were in the range from 621 to 3264 (for ISO) and from 12 ms to 16 ms (for $\Omega$) depending on the intensity of external light. The device-to-eye distance has been retrieved from the pre-calibrated position of focusing element within the camera imaging system. The measurement scheme is presented in Fig. 4(a). Alternatively, the camera-to-eye distance x is retrieved from at least one of the following data: (i) the position of focusing element within the camera imaging system; and/or (ii) the subject's face size in the captured video; and/or (iii) the measurement of the iris diameter; and/or (iv) the parallax effect estimated for images captured by different cameras; and/or (v) dedicated distance sensor based on ultrasound waves, infrared light, or LIDAR technique.

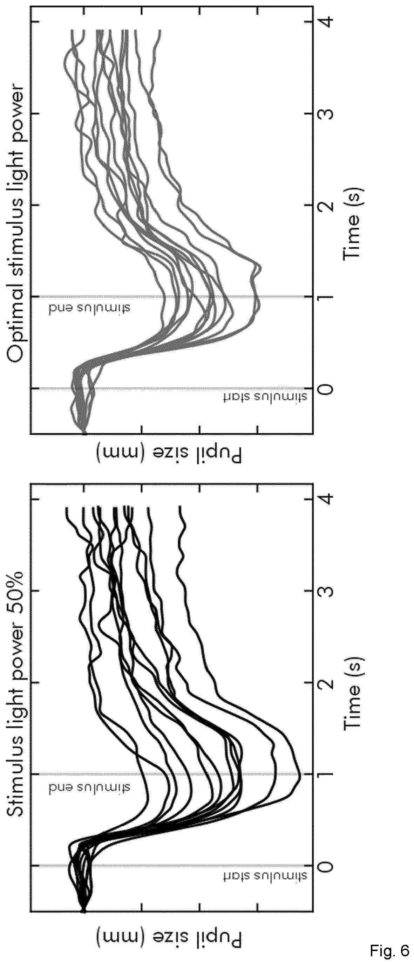

In order to confirm the reduction of variability it was made a series of measurements of several canonical pupillometric parameters CAMP, ACV, ADV, MCV, PDV parameters. The definition of parameters has been graphically presented in Fig. 4(b) and included in the glossary and definitions sections. All canonical pupillometric parameters of the volunteer eye have been measured for a plurality of external light intensities: 10 lux, 40 lux and 160 lux. Since the embodiment is aimed at reducing the variability resulting from non-controlled external light the measurements were made in a controlled device-to-eye distance yielding similar values of x = 130 mm for all measurement series. In the first measurement series the power of stimulus light intensity was always selected as 50% corresponding to the stimulus light luminance of 370 lux. In the second measurement series an optimal stimulus light power is calculated using a plot $L_{pow}$ (ISO, $\Omega$) presented in Fig. 6. The function $L_{pow}$ (ISO, $\Omega$) is defined implicitly as a numerical solution to the nonlinear equation:

$$\left[ Lum\left(x,\, L_{pow}(ISO,\, \Omega)\right) - Lum(x,\, p_0) \right] = \gamma \cdot \left( \frac{\Delta}{ISO \cdot \Omega} + Lum(x,\, p_0) \right), \quad (2)$$

where:

- $Lum(x,p)$ is a luminance of stimulus light source depending on its power level $p$ and distance from the source $x$ presented in Fig.1 (in the discussed embodiment $x$ = 130 mm).
- are light sensor parameters ISO and exposure time respectively

- $\gamma$ is the proportionality factor of the stimulation (in the discussed embodiment $\gamma$ = 2.5)
- $\Delta$ is the calibration constant relating light sensor settings to external light (in the discussed embodiment the measured constant $\Delta$ equals 455 s)
- $p_0$ is a power level of a stimulus light source before and after the stimulation (in the discussed embodiment $p_0$ = 0%) which might be optionally increased to facilitate pupil and iris detection

The left-hand side of the equation (1) defines the stimulus light resulting from switching the power level of the light stimulus source from $p_0$ to $L_{pow}(ISO, \Omega)$. The right-hand side of the equation is the external light reaching the subject's face before the stimulation multiplied by the proportionality factor $\gamma$. Thus, the formula ensures that the stimulation inducing the pupil light reaction is proportional to the light already illuminating the subject's pupil. The choice of specific proportionality factor $\gamma$ depends on the available optical power of the used stimulus light source as well as from the expected external light range.

The proportionality factor $\gamma$ in the optimized stimulus measurement series equalled 2.5, such that the optimized stimulus power yielded the illumination of 25 lux, 100 lux or 400 lux depending on the selected external light conditions.

Table 2. A set of pupillometric parameters measured in uncontrolled external light conditions (varying from 10 lux to 160 lux) using either fixed stimulus light intensity of 340 lux (corresponding to 50% of available stimulus light power) or using optimal stimulus light power.

| CAMP | | | |
|---|---|---|---|
| | 10 lux | 40 lux | 160 lux |
| Stimulus light power 50% | 1.38 mm | 0.94 mm | 0.59 mm |
| Optimal stimulus light power | 1.08 mm | 0.90 mm | 0.80 mm |

| ADV | | | |
|---|---|---|---|
| | 10 lux | 40 lux | 160 lux |
| Stimulus light power 50% | 0.74 mm/s | 0.53 mm/s | 0.37 mm/s |
| Optimal stimulus light power | 0.69 mm/s | 0.55 mm/s | 0.56 mm/s |

| ACV | | | |
|---|---|---|---|
| | 10 lux | 40 lux | 160 lux |
| Stimulus light power 50% | 1.44 mm/s | 1.59 mm/s | 0.83 mm/s |
| Optimal stimulus light power | 1.59 mm/s | 1.50 mm/s | 1.08 mm/s |

| MCV | | | |
|---|---|---|---|
| | 10 lux | 40 lux | 160 lux |
| Stimulus light power 50% | 3.72 mm/s | 3.56 mm/s | 1.90 mm/s |
| Optimal stimulus light power | 3.51 mm/s | 3.03 mm/s | 2.13 mm/s |

| PDV | | | |
|---|---|---|---|
| | 10 lux | 40 lux | 160 lux |
| Stimulus light power 50% | 1.40 mm/s | 0.88 mm/s | 0.52 mm/s |
| Optimal stimulus light power | 1.06 mm/s | 0.79 mm/s | 1.16 mm/s |

A set of five canonical pupillometric parameters CAMP, ADV, ACV, MCV, PDV, measured for fixed stimulus light power or optimal stimulus light power is presented in the Table 2. A reduction of variability is clearly visible for CAMP parameter, PDV parameter and ADV parameter. For CAMP parameter the fixed stimulus pupillary light response varied from 1.38 mm to 0.59 mm for the measurements performed with external light varying from 10 lux to 160 lux (resulting in 134% of relative CAMP change). In the same external conditions, the optimal stimulus light response varied from 1.08 mm to 0.80 mm (resulting in 35% of relative CAMP change). For PDV parameter the fixed stimulus pupillary light response varied from 1.40 mm/s to 0.52 mm/s for the measurements performed with external light varying from 10 lux to 160 lux (resulting in 169% of relative PDV change). In the same external conditions, the optimal stimulus light response varied from 1.06 mm/s to 0.79 mm/s (resulting in 34% of relative PDV change). For ADV parameter the fixed stimulus pupillary light response varied from 0.74 mm/s to 0.37 mm/s for the measurements performed with external light varying from 10 lux to 160 lux (resulting in 100% of relative ADV change). In the same external conditions, the optimal stimulus light response varied from 0.69 mm/s to 0.56 mm/s (resulting in 23% of relative ADV change). For ACV and MCV the variability of measurements is very similar due to the fact that both the fixed stimulus and optimized stimulus light resulted in pupil light reaction close to the saturation regime which induce the contraction of the pupil in the fastest possible way allowed by the iris sphincter.

In order to illustrate the reduction of variability of pupillary light response, Fig.6 presents a set of all collected pupillogram curves measured for fixed stimulus (left panel) and a set of all collected pupillogram curves measured for optimized stimulus (right panel). To reduce shot-to-shot measurement uncertainty in each external light conditions *i.e.* 10 lux, 40 lux and 160 lux five pupillogram curves were collected for fixed stimulus and five pupillogram curves were collected for optimized stimulus. This resulted in fifteen curves collected for fixed stimulus and fifteen curves collected for optimal stimulus.

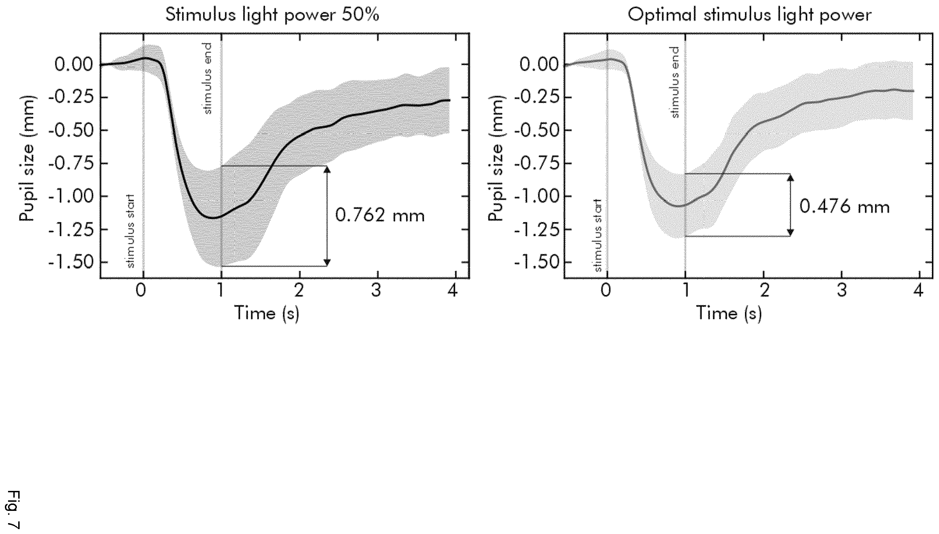

Finally, Fig. 7 presents an average of pupilogram series measured using either fixed stimulus light intensity (left panel) or using optimal stimulus light power (right panel). The uncertainty of the curve presented as a shaded region above and below the pupillogram curve has been characterized as a pointwise standard deviation calculated using all pupillometric curves collected for fixed or optimal stimulus light power. The uncertainty of the curve (calculated as two standard deviations) in the time instance when the light stimulation is turned off equals 0.762 mm for fixed stimulus light scenario and 0.476 mm for optimal stimulus light scenario. This shows the reduction of pupillary light response variability by approximately 38%.

### EMBODIMENT 3

### A method for reducing the variability of pupillary light response in visible-light pupillometry performed by a smartphone resulting from non-controlled environmental conditions, including device-to-eye distance and non-controlled external light

In the following embodiment a smartphone (Apple iPhone 13 Pro) is selected as a diagnostic or measurement device comprising a digital camera with an internal visible light source. The device is employed to collect the videos of pupillary light response induced by embedded flashlight with the video frame rate of 60 frames per second. After video capture each frame is fed into image segmentation or detection algorithm, estimating the pupil diameter and the iris diameter. Beforehand the image segmentation algorithm has been trained using an annotated dataset. The CAMP parameter is retrieved from the dynamics of pupillary light response also known as a pupillogram curve (see Fig. 2b).

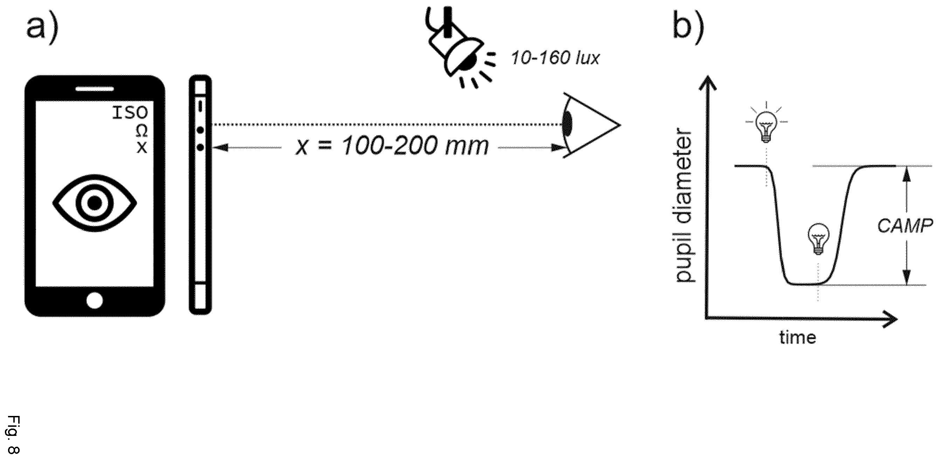

The reduction of the variability of pupillary light response is realised by adjusting the stimulus light intensity to the external light intensity. The detailed calibration curve of stimulus light source has been presented in Fig. 1. Before starting the measurement of pupillary light response, the settings of the light sensor are selected by the auto-exposure algorithm of the smartphone in order to bring the exposure value EV close to zero. The region of interest of the auto-exposure algorithm is the entire image. The values of ISO and $\Omega$ yielding the desired exposure value were retrieved and their values were in the range from 621 to 3264 (for ISO) and from 12 ms to 16 ms (for $\Omega$) depending on the intensity of external light. The device-to-eye distance has been retrieved from the pre-compensated position of focusing element within the camera imaging system. The measurement scheme is presented in Fig. 8(a). Alternatively, the camera-to-eye distance $x$ is retrieved from at least one of the following data: (i) the position of focusing element within the camera imaging system; and/or (ii) the subject's face size in the captured video; and/or (iii) the measurement of the iris diameter; and/or (iv) the parallax effect estimated for images captured by different cameras; and/or (v) dedicated distance sensor based on ultrasound waves, infrared light, or LIDAR technique.

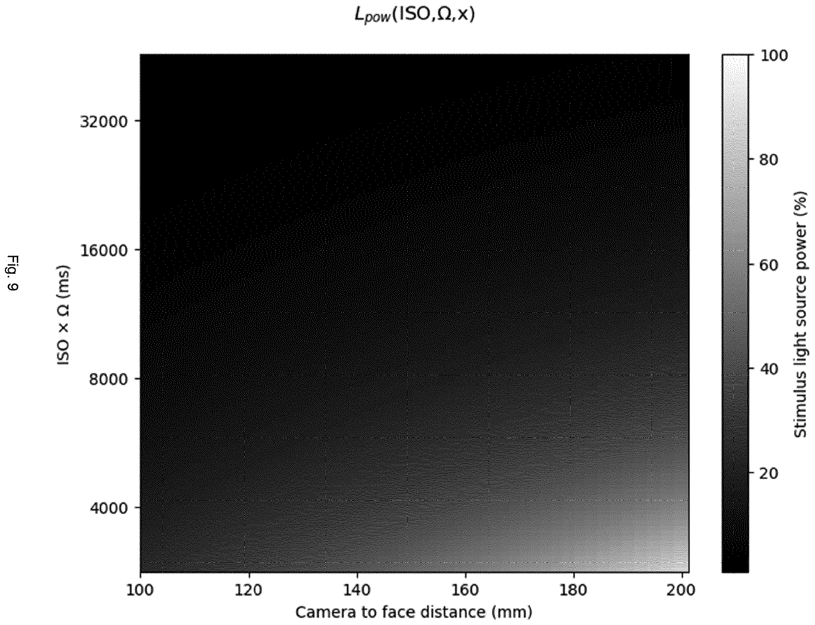

In order to confirm the reduction of variability it was performed a series of measurements of CAMP parameter which is the amplitude of pupil contraction expressed in millimetres induced by the stimulus light. The definition of CAMP has been graphically presented in Fig. 8(b). it was measured CAMP parameter of the volunteer eye for a plurality of distances between volunteer's eye and the diagnostic device: 100 mm, 150 mm and 200 mm, as well as for a plurality of external light intensities: 10 lux, 40 lux and 160 lux. In the first measurement series the power of stimulus light intensity was always selected as 50% corresponding to the stimulus light luminance of 633 lux, 306 lux or 183 lux, depending on the selected distance. In the second measurement series an optimal stimulus light power is calculated using a plot $L_{pow}$ (ISO, $\Omega$, $x$) presented in Fig. 9. The function $L_{pow}$ (ISO, $\Omega$, $x$) is defined implicitly as a numerical solution to the nonlinear equation:

$$\left[ Lum\left(x,\ L_{pow}(ISO,\ \Omega,\ x)\right) - Lum(x,\ p_0)\right] = \gamma \cdot \left(\frac{\Delta}{ISO\cdot\Omega} + Lum(x,\ p_0)\right),\ (3)$$

where:

- *Lum(x,p)* is a luminance of stimulus light source depending on its power level *p* and distance from the source *x* presented in Fig.1 (in the discussed embodiment *x* = 100 mm, 150 mm or 200 mm)
- *ISO, $\Omega$* are light sensor parameters ISO and exposure time respectively
- $\gamma$ is the proportionality factor of the stimulation (in the discussed embodiment $\gamma$ = 1.9)
- $\Delta$ is the calibration constant relating light sensor settings to external light (in the discussed embodiment the measured constant $\Delta$ equals 455 s)
- $p_0$ is a power level of a stimulus light source before and after the stimulation (in the discussed embodiment $p_0$ = 0%) which might be optionally increased to facilitate pupil and iris detection

The left-hand side of the equation (1) defines the stimulus light resulting from switching the power level of the light stimulus source from $p_0$ to $L_{pow}(ISO, \Omega, x)$. The right-hand side of the equation is the external light reaching the subject's face before the stimulation multiplied by the proportionality factor $\gamma$. Thus, the formula ensures that the stimulation inducing the pupil light reaction is proportional to the light already illuminating the subject's pupil. The choice of specific proportionality factor $\gamma$ depends on the available optical power of the used stimulus light source as well as from the expected external light range.

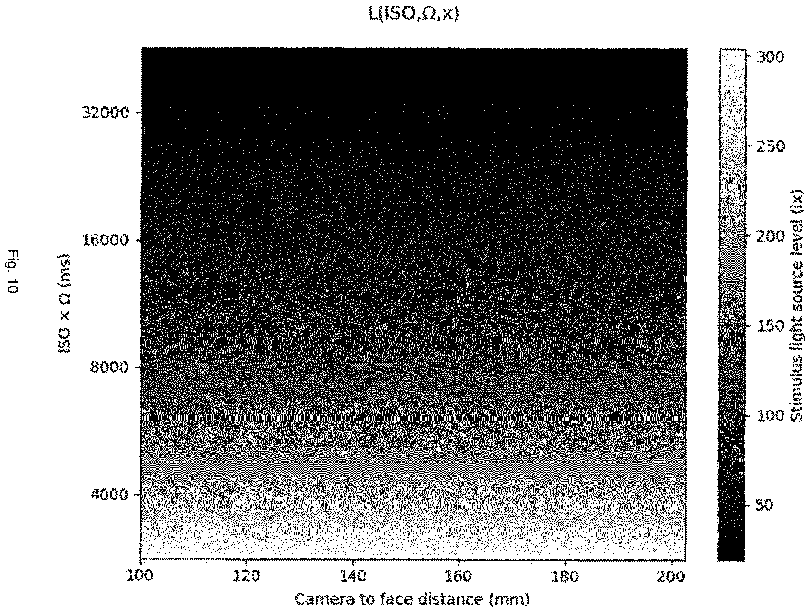

The proportionality factor $\gamma$ in the optimized stimulus measurement series equalled 1.9, meaning that the optimized stimulus power yielded the illumination of 19 lux, 76 lux or 304 lux depending on the selected external light conditions. The auxiliary matrix plot presenting the actual optimal stimulus illumination *L (ISO, $\Omega$, x)* has been presented in Fig. 10. Table 3. presents a list of optimal stimulus light powers for all distances and external light illuminations used in this particular embodiment.

Table 3. A set of optimal stimulus light powers for all distances and external light illuminations used in the embodiment 3.

| Optimal stimulus light power | 10 lux | 40 lux | 160 lux |
|---|---|---|---|
| x = 100 mm | 8.3% | 12.9% | 31.0% |
| x = 150 mm | 11.2% | 20.7% | 58.4% |
| x = 200 mm | 15% | 31.0% | 100% |

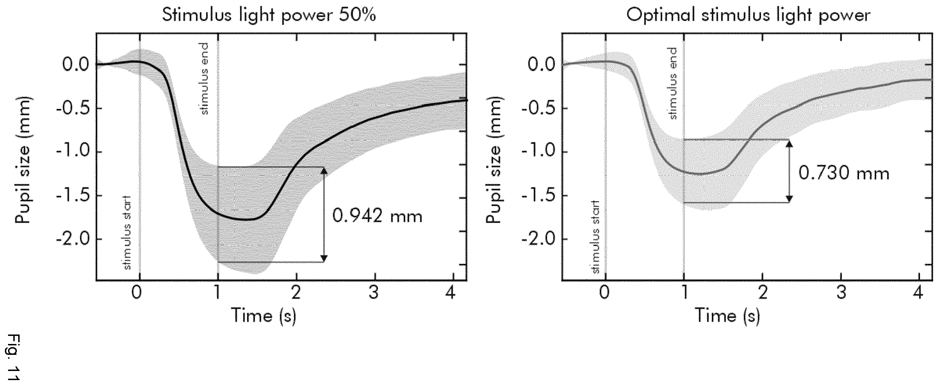

Fig. 11 presents an average of pupilogram series measured using either fixed stimulus light intensity (left panel) or using optimal stimulus light power (right panel). The uncertainty of the curve presented as a shaded region above and below the pupillogram curve has been characterized as a pointwise standard deviation calculated using all pupillometric curves collected for fixed or optimal stimulus light power. The uncertainty of the curve (calculated as two standard deviations) in the time instance when the light stimulation is turned off equals 0.942 mm for fixed stimulus light scenario and 0.730 mm for optimal stimulus light scenario. This shows the reduction of pupillary light response variability by approximately 23%.

**EMBODIMENT 4**

**A method for reducing the variability of pupillary light response in visible-light pupillometry performed by a smartphone using quality checks control before pupillometry measurement and post recording test**

In the current embodiment, apart from using an algorithm steps of reducing the variability of pupillary light response in visible-light during pupillometry measurement performed by a smartphone (or other mobile device) resulting from non-controlled environmental conditions, including device-to-eye distance as well as non-controlled external light, here it is proposed to further reduce the variability by applying a pre-recording quality checks and post-recording quality checks. The general purpose of the quality checks is to avoid unnecessary repetitions of a pupillometric recording, saving time of medical personnel, and to make sure that retrieved pupillometric parameters confidently reflect the current state of health of the patient. Reliable pupillometric measurements are essential for constant monitoring the health status of a patient by noticing any deviation in a pupillometric recordings of the patient, that could result from a neurodegenerative disease, brain trauma, concussion or other reasons.

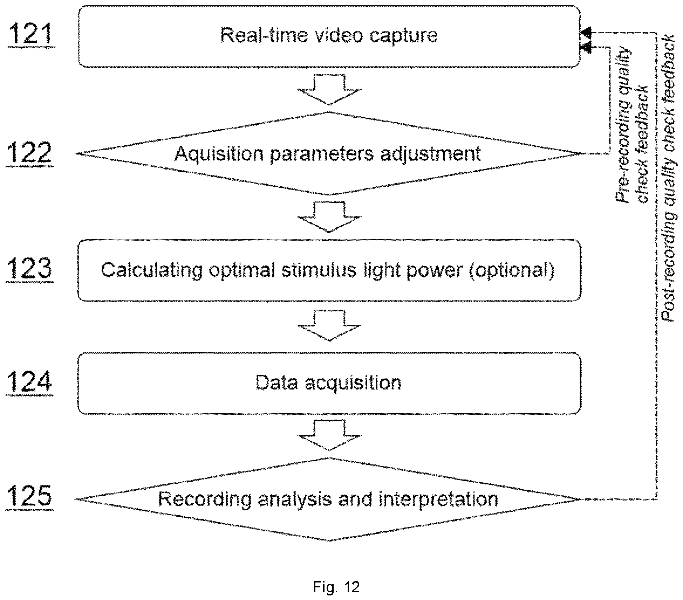

A general scheme for quality checks approach is presented in Fig. 12. Before recording the pupillometer curve a real-time video is captured (121) and pre-recording quality checks are applied (122) in order to eliminate the risk of repeating the measurement due to unacceptable video quality and avoid extreme measurement conditions which might heavily affect the retrieved pupillometric parameters such as CAMP, ADV, ACV, MCV, PDV. Pre-recordings checks are informing operator if the quality of the image is sufficient *i.e.* if the values of parameters such (*ISO, Ω, x*) do not exceed predefined acceptable range. If the pre-recording quality checks are not passed the operator is asked to change external light conditions, change the distance between diagnostic device and the eye or to tilt/pan the diagnostic device before the start of the measurement. This information is depicted in Fig. 12 as pre-recording quality check feedback. The pre-recording quality check might include:

- external light quality check - external illumination is a critical factor affecting both the video quality and pupil light reaction to the visible-light stimulus. To perform this quality check, one might rely on the combination of light sensor exposure time $\Omega$ and ISO value. In this embodiment, an inequality of $5.22 \text{ seconds} < ISO \times \Omega < \infty$ has been selected as a predefined range, which corresponded to the maximal acceptable external light of 300 lux. If pre-recording external light quality check is not passed the feedback is given to the operator to dim the external light before starting the measurement in order to improve properties of the recordings and avoid biased pupillary light response.
- distance quality check - is a test to establish a proper distance between mobile measurement device and an eye of patient is crucial to properly stimulate an eye as well as have enough coverage of pupil/eye in field of view of the light sensor. The optimal distance range is more than 5 cm and less than 30 cm, preferably more than 10 cm and less than 20 cm. If pre-recording distance quality check is not passed the feedback is given to the operator to move the diagnostic device closer/further to the patient eye.
- pupil size quality check - is an automated evaluation test detecting a presence of a pupil and/or an eye of a patient in the real-time video capture. To have a good quality recording, an eye and a pupil have to cover minimum number of pixels on a light sensor in a mobile device. If pre-recording pupil size quality check is not passed the feedback is given to the operator to move the diagnostic device closer to the patient eye or change the camera used for the measurement.
- stimulus light reflection position quality check - is an automated evaluation test to detect if the reflection of the stimulation light in the subject's eye is not located at the pupil/iris boundary. The undesired presence of the reflection at the aforementioned boundary might negatively affect the uncertainty of pupil diameter measurement. If pre-recording stimulus light reflection position quality check is not passed the feedback is given to the operator to tilt/pan the diagnostic device.

Only after the pre-recoding quality checks are passed, the pupillary light response is measured either using calculated optimal stimulus light power (123) or fixed stimulus light power. Apart from pre-recording quality checks yet another family of quality checks might be applied after the video capture (124) to (i) further confirm the quality of collected data and (ii) to make sure that the external conditions haven't changed during the measurement. The post-recording quality checks (125) might include:

- video undersaturation/oversaturation check - this check is designed to evaluate average pixel values in the captured video at the post-recording stage. The test enables to increase awareness of medical personnel that external light conditions has changed during the measurement and resulting pupillometric parameters might be negatively affected by this change. If post-recording video undersaturation/oversaturation check is not passed the feedback is given to the operator to increase/dim the external light and repeat the measurement.
- image sharpness check - this quality check is aimed at detecting any undesired movements of diagnostic device resulting from operator handshake or measurement distance instability which affect the sharpness of collected video. If post-recording sharpness check is not passed the feedback is given to the operator to repeat the measurement.
- pupil diameter measurement uncertainty check - regardless of applied method of pupil diameter retrieval from the captured video the uncertainty of the pupil diameter measurement (e.g., provided by the image segmentation algorithm) has to remain within the acceptable confidence level range over the entire timespan of the video. This test is aimed at verifying this data feature at the post-recording stage. If post-recording pupil diameter measurement uncertainty check is not passed the feedback is given to the operator to repeat the measurement.

If the post-recording quality checks are not passed the operator is asked to repeat the measurement. This information is depicted in Fig. 12 as post-recording quality check feedback.

Other quality tests are also possible to be included in the pre-recording as well as post-recording stage of the pupillometric measurements to maximise the probability of successful pupillometric parameters retrieval and reduce the variability of pupillary light response in a visible-light pupillometry performed by a diagnostic device in non-controlled environmental conditions.

## SUMMARY OF EMBODIMENTS

In summary, numerous modifications and variations of the present disclosure are possible in light of the above-described measurement method. It is therefore to be understood that within the scope of the appended claims, the embodiment may be practiced otherwise than as specifically described herein. For example, advantageous results may be achieved if the steps of the disclosed techniques were performed in a different sequence, if components in the disclosed systems were combined in a different manner, or if the components were replaced or supplemented by other components. The functions, processes, and algorithms described herein may be performed in hardware or software (computer-implemented invention) executed by hardware (smartphone or other device containing a camera with an adjustable focus), including computer processors and/or programmable processing circuits configured to execute program code and/or computer instructions to execute the functions, processes, and algorithms described herein. A processing circuit includes a programmed processor, as a processor includes circuitry. A processing circuit also includes devices such as an application specific integrated circuit (ASIC) and conventional circuit components arranged to perform the recited functions. The claimed features and associated function (technical effects) described herein may also be executed by various distributed components of a system. For example, one or more processors may execute described method.

## Claims

1.  A method for reducing the variability of pupillary light response in visible-light pupillometry implemented with the use of a mobile diagnostic device comprising a digital camera, an internal light source and a control unit, wherein the internal light source emits stimulus light in a visible spectrum with variable intensity based on the calculations of the control unit, wherein collected measured data are vulnerable to non-controllable external environmental conditions and stimulus light intensity emitted from the internal light source depends on camera light sensor settings and the camera-to-eye distance, said method comprising the following steps:

    a. the camera light sensor settings including sensor sensitivity ISO and sensor exposure time $\Omega$ are adjusted to yield the exposure value EV of the captured video in the range between the minimum exposure value and the maximum exposure value $[EV_{min}, EV_{max}]$, wherein the values of ISO and $\Omega$ yielding the expected exposure value are retrieved and stored for further use;
    b. the camera-to-eye distance $x$ is retrieved from at least one of the following data: (i) the position of focusing element within the camera imaging system; and/or (ii) the subject's face size in the captured video; and/or (iii) the measurement of the iris diameter; and/or (iv) the parallax effect estimated for images captured by different cameras; and/or (v) dedicated distance sensor based on ultrasound waves, infrared light, or LIDAR technique;
    c. the optimal stimulus light intensity $L$ is calculated by the control unit using the function $L(x)$ or $L(ISO, \Omega)$ or $L(ISO, \Omega, x)$, wherein the exact values of function arguments are retrieved from the steps a and b, while the optimal stimulus light intensity $L$ is either expressed in luminance units or in optimal stimulus light power varying from 0% to 100% with the latter case referred to as $L_{pow}(x)$, $L_{pow}(ISO, \Omega)$ and $L_{pow}(ISO, \Omega, x)$ respectively.

2.  The method according to claim 1, **characterized in that** it further comprises step:
    d. a video of an eye is captured, including the time period before the eye is illuminated with the optimal stimulus light intensity calculated in step c, the time period when the eye is illuminated with said stimulus and the time period after the eye is illuminated with said stimulus, and afterwards a pupillometric curve is reconstructed by feeding the measured video frame-by-frame data to image segmentation algorithm learned beforehand by means of an annotated dataset.

3.  The method according to claims 1 or 2, **characterized in that** the optimal stimulus light intensity $L$ calculated using the function $L(x)$ or $L(ISO, \Omega)$ or $L(ISO, \Omega, x)$ or alternatively $L_{pow}(x)$, $L_{pow}(ISO, \Omega)$ or $L_{pow}(ISO, \Omega, x)$, respectively, increases monotonically in response to increasing external light.

4.  The method according to any one of claims 1 to 3, **characterized in that** the optimal stimulus light intensity $L$ calculated using the function $L(x)$ or $L(ISO, \Omega)$ or $L(ISO, \Omega, x)$ or alternatively $L_{pow}(x)$, $L_{pow}(ISO, \Omega)$ or $L_{pow}(ISO, \Omega, x)$, respectively, is proportional to the intensity of external light with a proportionality factor $\gamma$.

5.  The method according to any one of claims 1 to 4, **characterized in that** the settings of the camera light sensor which yield the exposure value EV of the captured video in the range of $[EV_{min}, EV_{max}]$ are based on either the entire image or the selected region of interest within said image, such as the area of subject's iris, sclera or forehead.

6.  The method according to any one of claims 1 to 5, **characterized in that** the settings of the camera light sensor which yield the exposure value EV of the captured video in the range of [$EV_{min}$, $EV_{max}$] are adjusted manually by a diagnostic device operator or automatically by an auto-exposure algorithm.

7. The method according to any one of claims 1 to 6, **characterized in that** a family of functions $L_n(ISO, \Omega, x)$ is used for the calculation of the optimal stimulus light intensity $L$ with a specific function selection depending on the iris colour, skin colour or hair colour of the measured subject.

8. The method according to any one of claims 1 to 7, **characterized in that** the values of the function $L(ISO, \Omega, x)$ or the family of functions $L_n(ISO, \Omega, x)$ are pre-calculated and stored on a computer readable storage medium.

9. The method according to any one of claims 1 to 8, **characterized in that** the diagnostic device is used, which is equipped with a dedicated light sensor, whose readout $I_{ext}$ is used to calculate optimal stimulus light intensity $L$ by using the function $L(x)$ or $L_{pow}(x)$, respectively, which additionally depends also on $I_{ext}$.

10. The method according to any one of claims 1 to 9, **characterized in that** the range [$EV_{min}$, $EV_{max}$] is defined as [$\eta$ -0.5, $\eta$+0.5], where $\eta$ is a real number belonging to the interval [-6, 21].

11. The method according to claim 10, **characterized in that** the range [$EV_{min}$, $EV_{max}$] is defined as [$\eta$-0.1, $\eta$+0.1], where $\eta$ is a real number belonging to the interval [-6, 21].

12. The method according to any one of claims 1 to 11, **characterized in that** the optimal stimulus light power is calculated using a plot $L_{pow}(x)$ or $L_{pow}(ISO, \Omega)$ or $L_{pow}(ISO, \Omega, x)$, wherein the function $L_{pow}(x)$ or $L_{pow}(ISO, \Omega)$ or $L_{pow}(ISO, \Omega, x)$ is defined implicitly as a numerical solution to the nonlinear equation:

$$\left[Lum\left(x, L_{pow}(x)\right) - Lum(x, p_0)\right] = \gamma \cdot \left(\frac{\Delta}{ISO \cdot \Omega} + Lum(x, p_0)\right)$$

Or

$$\left[Lum\left(x, L_{pow}(ISO, \Omega)\right) - Lum(x, p_0)\right] = \gamma \cdot \left(\frac{\Delta}{ISO \cdot \Omega} + Lum(x, p_0)\right)$$

Or

$$\left[Lum\left(x, L_{pow}(ISO, \Omega, x)\right) - Lum(x, p_0)\right] = \gamma \cdot \left(\frac{\Delta}{ISO \cdot \Omega} + Lum(x, p_0)\right)$$

13. The method according to any one of claims 1 to 12, **characterized in that** after the steps a and b a pre-recording quality check is performed based on the values of parameters ($ISO, \Omega, x$) retrieved in these steps and if the values of said parameters are out of a predefined range - feedback information is given to a diagnostic device operator to modify the external conditions before the start of the measurement.

14. The method according to any one of claims 1 to 13, **characterized in that** an additional pre-recording quality check is performed based on the diameter of the pupil in the video and the position of the light source reflection on the eyeball and if these image features are out of the predefined range - feedback information is given to an operator to tilt/pan or move the diagnostic device before the start of the measurement.

15. The method according to any one of claims 1 to 14, **characterized in that** after completing the method steps an additional post-recording quality check is performed based on the sharpness and brightness of the video or pupil diameter measurement uncertainty and if these parameters are out of predefined range - feedback information is given to an operator to repeat the measurement.

$$Lum(x,p) = I(p)+A(p)/x^{n(p)}$$

$I(p) = 6.88-0.271p+0.003p^2$
$A(p) = -1621+44915p+88.595p^2$
$n(p) = 1.78-4.435*10^{-6}p^2$

Fig. 1

Fig. 2

Fig. 3

Fig. 4

EP 4 480 391 A1

Fig. 5

Fig. 6

Fig. 7

Fig. 8

$L_{pow}(\text{ISO},\Omega,x)$

Fig. 9

Fig. 10

Fig. 11

121 — Real-time video capture

122 — Aquisition parameters adjustment

123 — Calculating optimal stimulus light power (optional)

124 — Data acquisition

125 — Recording analysis and interpretation

*Pre-recording quality check feedback*

*Post-recording quality check feedback*

Fig. 12

Europäisches
Patentamt

European
Patent Office

Office européen
des brevets

# EUROPEAN SEARCH REPORT

Application Number

**EP 23 46 1609**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/034951 A1 (BIOTRILLION INC [US]) 25 February 2021 (2021-02-25) * paragraphs [0013], [0042], [0050], [0051], [0063], [0084], [0086] * | 1-15 | INV. A61B3/14 A61B3/11 |
| X | US 2022/233072 A1 (SIMINOU KAMRAN [US]) 28 July 2022 (2022-07-28) * paragraphs [0015], [0019], [0026], [0027] * | 1-15 | |

TECHNICAL FIELDS
SEARCHED (IPC)

A61B

The present search report has been drawn up for all claims

| Place of search | Date of completion of the search | Examiner |
|---|---|---|
| Munich | 16 November 2023 | Martelli, Luca |

EPO FORM 1503 03.82 (P04C01)

**ANNEX TO THE EUROPEAN SEARCH REPORT
ON EUROPEAN PATENT APPLICATION NO.**                EP 23 46 1609

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2023

| Patent document cited in search report | Publication date | Patent family member(s) | | Publication date |
|---|---|---|---|---|
| WO 2021034951 A1 | 25-02-2021 | US | 2022287607 A1 | 15-09-2022 |
| | | WO | 2021034931 A1 | 25-02-2021 |
| | | WO | 2021034951 A1 | 25-02-2021 |
| US 2022233072 A1 | 28-07-2022 | US | 2022233072 A1 | 28-07-2022 |
| | | WO | 2020168009 A1 | 20-08-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018222897 A1
- US 10070787 B2
- US 10034605 B2
- WO 2021034951 A1
- US 0000339 A1
- US 10506165 B2
- US 11003936 B2